(19) Europäisches Patentamt
European Patent Office
Office européen des brevets

(11) **EP 3 908 253 B1**

(12) **EUROPÄISCHE PATENTSCHRIFT**

(45) Veröffentlichungstag und Bekanntmachung des Hinweises auf die Patenterteilung:
**14.08.2024 Patentblatt 2024/33**

(21) Anmeldenummer: **20700679.2**

(22) Anmeldetag: **10.01.2020**

(51) Internationale Patentklassifikation (IPC):
*A61K 9/00* (2006.01)   *A61K 47/10* (2017.01)
*A61K 47/14* (2017.01)   *A61K 47/22* (2006.01)
*A61K 47/32* (2006.01)   *A61K 47/36* (2006.01)
*A61K 47/44* (2017.01)   *A61K 31/352* (2006.01)

(52) Gemeinsame Patentklassifikation (CPC):
**A61K 9/006; A61K 31/352; A61K 47/10;
A61K 47/14; A61K 47/22; A61K 47/26;
A61K 47/32; A61K 47/36; A61K 47/44;
A61P 15/02; A61P 19/02; A61P 25/00;
A61P 25/02; A61P 25/04; A61P 25/06;** (Forts.)

(86) Internationale Anmeldenummer:
**PCT/EP2020/050569**

(87) Internationale Veröffentlichungsnummer:
**WO 2020/144345 (16.07.2020 Gazette 2020/29)**

(54) **ORALER DÜNNFILM**

ORAL THIN FILM

FILM MINCE D'HYGIÈNE BUCCO-DENTAIRE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.01.2019 DE 102019100483**

(43) Veröffentlichungstag der Anmeldung:
**17.11.2021 Patentblatt 2021/46**

(73) Patentinhaber: **LTS LOHMANN Therapie-Systeme AG
56626 Andernach (DE)**

(72) Erfinder:
 • **BAUER, Marius
   56626 Andernach (DE)**
 • **LINN, Michael
   55596 Waldböckelheim (DE)**
 • **EMGENBROICH, Marco
   53359 Rheinbach (DE)**
 • **SCHMITZ, Christoph
   56598 Rheinbrohl (DE)**
 • **MÜLLER, Markus
   53840 Troisdorf (DE)**

(74) Vertreter: **Meissner Bolte Partnerschaft mbB
Patentanwälte Rechtsanwälte
Postfach 86 06 24
81633 München (DE)**

(56) Entgegenhaltungen:
WO-A1-03/101357     WO-A1-2018/211388
WO-A1-2020/014776   WO-A2-02/064109
WO-A2-03/105800     US-A1- 2016 051 510

(52) Gemeinsame Patentklassifikation (CPC): (Forts.)
**A61P 25/14; A61P 25/16; A61P 25/30;**
**A61P 25/36; A61P 27/06**

(52) Gemeinsame Patentklassifikation (CPC): (Forts.)
**A61P 25/14; A61P 25/16; A61P 25/30;**
**A61P 25/36; A61P 27/06**

**Beschreibung**

Die vorliegende Erfindung betrifft einen oralen Dünnfilm zur Verabreichung von aktiven Wirkstoffen aus der Gruppe der Cannabinoide, sowie eine Verabreichungsform für diese Wirkstoffe, Verfahren zu deren Herstellung und deren Verwendung als Arzneimittel.

Die orale Verabreichung von aktiven Wirkstoffen aus der Gruppe der Cannabinoide in Form von Kapseln, Tabletten, Pillen, anderen festen oralen Darreichungsformen oder in Form von oral zu verabreichenden flüssigen Zubereitungen ist aus mehreren Gründen nachteilig.

Zum einen erfolgt die Resorption des Wirkstoffs im Gastrointestinaltrakt. Dadurch wird der Zeitpunkt des Wirkungseintritts verzögert, was einem schnellen Wirkungseintritt entgegensteht. Zum anderen werden die Wirkstoffe aus der Gruppe der Cannabinoide bei oraler Aufnahme während der Magen-Darm-Passage unter dem Einfluss von Säuren bzw. Enzymen zumindest teilweise abgebaut und/oder inaktiviert, sodass nur ein Teil der eingenommenen Dosis tatsächlich als aktiver Wirkstoff wirken kann. Außerdem wird nach oraler Verabreichung ein bedeutender Teil des Wirkstoffs bereits während der ersten Leberpassage metabolisiert ("first-pass" Effekt).

Die vorstehend beschreibenden Probleme können durch Verabreichung eines aktiven Wirkstoffs aus der Gruppe der Cannabinoide mittels eines oralen Dünnfilms überwunden werden. Orale Dünnfilme, auch transmukosale Verabreichungssysteme genannt, sind dünne wirkstoffhaltige Filme auf Polymerbasis, die, wenn sie auf eine Schleimhaut, insbesondere die Mundschleimhaut, aufgebracht werden, den Wirkstoff direkt in diese abgeben. Diese Darreichungssysteme haben den Vorteil, dass der Wirkstoff größtenteils durch die Schleimhaut resorbiert wird und damit der "First-Pass Metabolismus", der bei der konventionellen Darreichungsform eines Wirkstoffs in Tablettenform beachtet werden muss, vermieden wird.

Die Verabreichung von aktiven Wirkstoffen aus der Gruppe der Cannabinoide in Form von oralen Dünnfilmen ist aus dem Stand der Technik bekannt.

So offenbart die WO 03/105800 A2 einen oralen Dünnfilm zur Verabreichung von Cannabinoiden, bei denen der aktive Wirkstoff in einer hydrophilen, wasserlöslichen Matrix eingebettet ist.

Die CA 2922959 A1 offenbart einen oralen Dünnfilm zur Verabreichung von Cannabinoiden, wobei die aktiven Wirkstoffe in Form von Nanomizellen, umfassend den Wirkstoff in wässriger Lösung, in einem äußeren filmbildenden Polymer vorliegen. Die WO 02/064109 offenbart orale Dünnfilme beinhaltend THC und 0,1 Gew.-% Tocopherol.

Zudem sind aus den USA frei und kommerziell erhältliche Cannabis-Strips bekannt, wobei ein THC-Extrakt neben Geschmacksstoffen in einer hydrophilen Polymermatrix eingebettet vorliegt.

Die aus dem Stand der Technik bekannten Darreichungsformen von Cannabinoiden in Form von oralen Dünnfilmen oder Strips haben jedoch den Nachteil, dass die Wirkstoffe aus der Gruppe der Cannabinoide relativ anfällig bezüglich Oxidation und somit relativ instabil sind, wenn sie grob-dispers in ein wasserlösliches Polymer eingearbeitet werden. Insbesondere Tetrahydrocannabinol (THC) liegt, wenn es in einer hydrophilen Matrix eingebettet ist, in wachsartiger/harziger Form als separate Phase vor, was besonders nachteilig bezüglich dem oxidativem Abbau des Wirkstoffs und der Stabilität des oralen Dünnfilms bezüglich Phasentrennung ist. Durch die Empfindlichkeit der Wirkstoffe aus der Gruppe der Cannabinoide gegenüber oxidativem Abbau sind die aus dem Stand der Technik bekannten oralen Dünnfilme insbesondere hinsichtlich ihrer Lagerungsstabilität nachteilig. Zudem haben die Darreichungsformen in Form von Cannabis-Strips den Nachteil, dass diese Strips relativ dick und damit unflexibel sind, was den Tragekomfort im Mundraum beeinträchtigt.

Die Aufgabe der vorliegenden Erfindung besteht darin, vorstehend genannte Nachteile des Standes der Technik zu beheben. Insbesondere besteht die Aufgabe der vorliegenden Erfindung darin, einen oralen Dünnfilm bzw. ein transmukosales Verabreichungssystem bereitzustellen, bei dem die aktiven Wirkstoffe aus der Gruppe der Cannabinoide chemisch stabil, d.h. insbesondere vor oxidativem Abbau geschützt, vorliegen. Insbesondere soll der orale Dünnfilm über eine längere Zeit gelagert werden können, ohne das der Wirkstoff aus der Gruppe der Cannabinoide signifikant, insbesondere oxidativ, abgebaut wird. Zudem soll der orale Dünnfilm einfach und kostengünstig herzustellen sein.

Obige Aufgabe wird durch einen oralen Dünnfilm bzw. ein transmukosales Verabreichungssystem nach Anspruch 1 gelöst, der bzw. das eine äußere hydrophile Phase, die mindestens ein hydrophiles Polymer enthält, und eine innere hydrophobe Phase, die mindestens eine hydrophobe Substanz und mindestens einen pharmazeutisch aktiven Wirkstoff ausgewählt aus der Gruppe der Cannabinoide enthält, wobei der orale Dünnfilm zusätzlich Vitamin E in einer Menge von 5 bis 20 Gew.-%, bezogen auf das Gesamtgewicht des oralen Dünnfilms, umfasst, wobei Vitamin E mindestens eine Verbindung ausgewählt aus $\alpha$-Tocopherol, $\beta$-Tocopherol, $\gamma$-Tocopherol, $\delta$-Tocopherol, $\alpha$-Tocotrienol, $\beta$-Tocotrienol, $\gamma$-Tocotrienol, $\delta$-Tocotrienol und/oder $\alpha$-Tocopherol-acetat umfasst.

Ein solches System hat den Vorteil, dass der aktive Wirkstoff aus der Gruppe der Cannabinoide in einer hydrophoben Umgebung vorliegt, die in der hydrophilen Phase stabilisiert vorliegt, was eine Phasentrennung in dem oralen Dünnfilm weitgehend verhindert. Zudem ist der Wirkstoff aus der Gruppe der Cannabinoide, wenn er in einer hydrophoben Umgebung vorliegt, deutlich besser gegenüber oxidativem Abbau stabilisiert. Zudem liegt der aktive Wirkstoff aus der Gruppe der Cannabinoide in einer hydrophoben Umgebung in Lösung vor, was sich auch positiv auf die Resorption auswirken kann. Durch das Lösen des aktiven Wirkstoffs aus der Gruppe der Cannabinoide in einer hydrophoben Substanz werden außerdem die Verarbeitung des Wirkstoffs und die Herstellung des oralen Dünnfilms deutlich erleichtert.

Der erfindungsgemäße orale Dünnfilm liegt vorzugsweise als Öl-in-Wasser Emulsion vor. Diese Öl-in-Wasser Emulsion wird dabei vorzugsweise durch den mindestens einen Emulgator stabilisiert.

Es hat sich auch gezeigt, dass Vitamin E, vorzugsweise in einer Menge von mindestens etwa 1 Gew.-% (beansprucht sind 5-20 Gew.-%), wobei das Vitamin E selbst keine emulgierenden Eigenschaften aufweist, sondern als zusätzliches Lösungsmittel für den mindestens einen Wirkstoff aus der Gruppe der Cannabinoide dient, die innere hydrophobe Phase enthaltend den mindestens einen Wirkstoff aus der Gruppe der Cannabinoide auch in der hydrophilen Phase stabilisieren kann. Vitamin E, bevorzugt in einer Menge von mindestens etwa 1 Gew.-% (beansprucht sind 5-20 Gew.-%), kann somit zusätzlich zu mindestens einem Emulgator eingesetzt werden.

Durch die Hydrophobizität des Vitamin E liegt Vitamin E zusammen mit der mindestens einen hydrophoben Substanz und dem mindestens einen Wirkstoff aus der Gruppe der Cannabinoide vorzugsweise in der inneren hydrophoben Phase vor. Der Begriff "hydrophobe Substanz" soll dabei ausdrücklich nicht die hydrophoben Substanz Vitamin E umfassen.

Vitamin E umfasst die chemischen Verbindungen o-Tocopherol, β-Tocopherol, γ-Tocopherol, δ-Tocopherol und o-Tocotrienol, β-Tocotrienol, γ-Tocotrienol und δ-Tocotrienol.

Als pharmazeutisch akzeptables Derivat von Vitamin E ist insbesondere α-Tocopherol-acetat bevorzugt.

In einer bevorzugten Ausführungsform des erfindungsgemäßen oralen Dünnfilms liegt der aktive Wirkstoff aus der Gruppe der Cannabinoide im Wesentlichen in der inneren hydrophoben Phase des Systems vor. Unter im Wesentlichen wird verstanden, dass der aktive Wirkstoff aus der Gruppe der Cannabinoide zu mehr als etwa 80 Gew.-%, bevorzugt mehr als etwa 85 Gew.-% und besonders bevorzugt zu mehr als etwa 90 Gew.-%, ganz besonders bevorzugt zu mehr als etwa 95 Gew.-% und noch mehr bevorzugt zu mehr als etwa 99 Gew.-%, bezogen auf die Gesamtmenge an aktivem Wirkstoff aus der Gruppe der Cannabinoide in dem oralen Dünnfilm, in der inneren hydrophoben Phase des oralen Dünnfilms vorliegt.

Dies hat den Vorteil, dass der aktive Wirkstoff aus der Gruppe der Cannabinoide im Wesentlichen in der inneren Phase gelöst vorliegt und somit chemisch und/oder physikalisch stabiler vorliegt. Liegt der aktive Wirkstoff aus der Gruppe der Cannabinoide zu weniger als 80 Gew.-% in der inneren hydrophoben Phase vor, so hat dies den Nachteil, dass zu viel des aktiven Wirkstoffs aus der Gruppe der Cannabinoide in der äußeren hydrophilen Phase vorliegt, was den oxidativen Abbau des Wirkstoffs begünstigt.

Der Begriff Cannabinoide steht für eine Sammelbezeichnung für aus Cannabis-Arten isolierbaren terpenoiden Inhaltsstoffen mit 21 Kohlenstoff-Atomen, hauptsächlich Benzopyran-Derivaten, und deren (semi)synthetische Derivaten. Es sind mehr als 70 natürlich vorkommende Cannabinoide bekannt, von denen einige psychotrope und andere pharmakologische Effekte haben.

Eine nicht abschließende Liste von Beispielen für Cannabinoide umfasst: Cannabichromanon, Cannabichromen, Cannabicoumaronon, Cannabicyclol, Cannabidiol, Cannabidivarin, Cannabidivarinsäure, Cannabifuran, Cannabinodiol, Cannabinol, Cannabinolsäure, Cannabitriol, Cannabivarichromen, Cannabivarin, Δ8 -Tetrahydrocannabinol, Δ9 -Tetrahydrocannabinol. Die Wirkstoffe aus der Gruppe der Cannabinoide können natürlichen, teilsynthetischen oder synthetischen Ursprungs sein.

Als synthetisch hergestelltes Cannabinoid ist außerdem R-(6a,10a)-Δ9-Tetrahydrocannabinol zur Verabreichung in dem erfindungsgemäßen oralen Dünnfilm geeignet.

Es kommen auch Cannabis-Extrakte und Cannabis-Öle, insbesondere Extrakte und Öle von *Cannabis sativa* oder *Cannabis indica,* in Betracht. Cannabis-Extrakte bzw. -Öle enthalten als pharmakologisch aktive Inhaltsstoffe u.a. Tetrahydrocannabinol (überwiegend Δ9-Tetrahydrocannabinol, in geringerem Anteil Δ8-Tetrahydro-cannabinol), Cannabidiol, Cannabinol und Cannabichromen.

Besonders bevorzugt enthält der erfindungsgemäße orale Dünnfilm mindestens einen pharmazeutisch aktiven Wirkstoff ausgewählt aus der Gruppe der Cannabinoide Tetrahydrocannabinol (THC), bevorzugt Δ8 -Tetrahydrocannabinol, Δ9 -Tetrahydrocannabinol oder R-(6a,10a)-Δ9 -Tetrahydrocannabinol, Cannabinol, Cannabidiol und/oder Cannabichromen.

Der erfindungsgemäße orale Dünnfilm ist außerdem vorzugsweise dadurch gekennzeichnet, dass die Menge des mindestens einen pharmazeutisch aktiven Wirkstoffs ausgewählt aus der Gruppe der Cannabinoide etwa 1 bis 30 Gew.-%, bevorzugt etwa 2 bis 25 Gew.-%, besonders bevorzugt etwa 5 bis 20 Gew.-% und noch mehr bevorzugt etwa 8 bis 12 Gew.-%, bezogen auf das Gesamtgewicht des oralen Dünnfilms, beträgt.

Das hydrophile Polymer in der äußeren hydrophilen Phase ist ein Polymer, das polare oder geladene Gruppen enthält. Diese Gruppen können nicht-ionisch, anionisch, kationisch oder zwitterionisch sein. Hydrophile Polymere sind in der Regel wasserlöslich.

Besonders bevorzugt ist das hydrophile Polymer in der äußeren hydrophilen Phase ein hydrophiles und wasserlösliches Polymer. Wasserlösliche Polymere umfassen chemisch sehr unterschiedliche, natürliche oder synthetische Polymere, deren gemeinsames Merkmal ihre Löslichkeit in Wasser oder wässrigen Medien ist. Voraussetzung dafür ist, dass diese Polymere eine für die Wasserlöslichkeit ausreichende Anzahl an hydrophilen Gruppen besitzen und nicht vernetzt sind.

Das hydrophile Polymer der äußeren hydrophilen Phase des erfindungsgemäßen oralen Dünnfilms umfasst vorzugsweise ein hydrophiles Polymer ausgewählt aus der Gruppe bestehend aus Stärke und Stärkederivaten, Dextran, Cellulose und Cellulosederivaten, wie Carboxymethylcellulose, Hydroxypropylcellulose, Hydroxyethylcellulose, Hydroxypropylmethylcellulose, Hydroxypropylethylcellulose, Natrium-Carboxymethylcellulose, Ethyl- oder Propylcellulose, Polyacrylsäure, Polyacrylat, Polyvinylpyrrolidon, Polyetylenglycol/PolyvinylalkoholCopolymer, Polyvinylalkohol, Polyethylenoxid-Polymeren, Polyethylenoxid/Polyethylenglykol-Copolymeren, Polyacrylamid, Polyethylenglykol, Gelatine, Kollagen, Alginat, Pectin, Pullulan, Traganth, Chitosan, Alginsäure, Arabinogalactan, Galactomannan, Agar-Agar, Agarose, Carrageen, Schellack, natürliche Gummen und/oder Copolymeren davon.

In einer besonders bevorzugten Ausführungsform umfasst das mindestens eine hydrophile Polymer Pullulan, Polyvinylalkohol, Polyvinylpyrrolidon, ein Cellulosederivat, insbesondere Hydroxypropylmethylcellulose und/oder Copolymere davon. Besonders bevorzugt ist der Einsatz von Polyvinylalkohol.

Diese hydrophilen Polymere haben den Vorteil, dass sie getrocknet einen dünnen stabilen Film bilden, der sich bei Applikation auf der Schleimhaut in einem pharmazeutisch akzeptablen Zeitraum auflöst und damit den Wirkstoff freigibt, was den Vorteil einer relativ schnellen Verfügbarkeit des Wirkstoffs sowie einer rückstandlosen Darreichung des Wirkstoffs hat.

Insbesondere Polyvinylalkohol hat den Vorteil, dass Polyvinylalkohol selbst emulgierende Eigenschaften aufweist und somit den oralen Dünnfilm bezüglich Phasentrennung stabilisieren kann.

Die innere hydrophobe Phase des erfindungsgemäßen oralen Dünnfilms umfasst mindestens eine hydrophobe Substanz.

Unter einer hydrophoben Substanz wird eine Substanz verstanden, deren logP-Wert größer als etwa 1, bevorzugt größer als etwa 1,5, besonders bevorzugt größer als etwa 2 ist.

Der n-Octanol-Wasser-Verteilungskoeffizient $K_{ow}$ (auch Schreibweisen wie Octanol/Wasser-Verteilungskoeffizient sind gebräuchlich und korrekt) ist ein dem Fachmann bekannter dimensionsloser Verteilungskoeffizient, der das Verhältnis der Konzentrationen einer Chemikalie in einem Zweiphasensystem aus n-Octanol und Wasser angibt und damit ein Maß für die Hydrophobizität bzw. Hydrophilität eines Stoffes ist. Der logP-Wert ist der dekadische Logarithmus des n-Octanol-Wasser-Verteilungskoeffizienten $K_{ow}$. Dabei gilt:

$$K_{ow} = P = \frac{c_0^{Si}}{c_w^{Si}} \qquad \text{und} \qquad \log P = \log \frac{c_0^{Si}}{c_w^{Si}} = \log c_o^{Si} - c_w^{Si}$$

mit $c_o^{Si}$ = Konzentration einer Chemikalie in der octanolreichen Phase und
$c_w^{Si}$ = Konzentration einer Chemikalie in der wasserreichen Phase.
$K_{ow}$ ist größer als eins, wenn eine Substanz besser in fettähnlichen Lösungsmitteln wie n-Octanol löslich ist, und kleiner als eins wenn sie besser in Wasser löslich ist. Entsprechend ist log P positiv für lipophile und negativ für hydrophile Substanzen. Da es sich bei den Cannabinoiden um hydrophobe Substanzen handelt, lösen sich die Cannabinoide bevorzugt in einer hydrophoben Substanz mit einen logP-Wert von größer als etwa 1, bevorzugt von größer als etwa 1,5, besonders bevorzugt von größer als etwa 2.

Vorteilhaft wird die hydrophobe Substanz ausgewählt aus der Gruppe pharmazeutisch akzeptabler hydrophober Substanzen, wie Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisonanoat, 2-Ethylhexylpalmitat, 2-Ethylhexyllaurat, 2-Hexyldecylstearat, Dialkylethern, Alkoholen, Fettsäuretriglyceriden, wie Triglycerinestern gesättigter und/oder ungesättigter Alkancarbonsäuren, Glycerolmono- und dioleaten, synthetischen, halbsynthetischen und natürlichen Ölen, wie Olivenöl, Mandelöl, Avocadoöl, Sonnenblumenöl, Sojaöl, Erdnussöl, Rapsöl, Palmöl, Kokosöl, Palmkernöl, 2-Octyldodecylpalmitat, Ethyloleat, Oleyloleat, Oleylecurat, Erucyloleat sowie synthetischen, halbsynthetischen und natürlichen Gemischen solcher Ester, mittelkettigen Triglyceriden, Paraffinöl, Squalen oder Squalan, Fettalkoholen mit 6 bis 18 Kohlenstoffatomen in geraden Ketten und/oder Säuren aus der Gruppe Laurin-, Palmitin-, Myristin-, Aradidon, Öl-, Linolen- und Linolsäure, Methylsalicylat, Tributylcitrat, Triethylcitrat, Eucalyptol, 1,2-Propandiol und/oder Methylsalicylat.

Besonders bevorzugt sind mittelkettige Triglyceride, insbesondere Isopropylmyristat, Fettsäuren, insbesondere Laurinsäure, und/oder Mischungen davon.

Der Begriff hydrophobe Substanz umfasst insbesondere nicht Vitamin E gemäß Anspruch 1.

Der erfindungsgemäße orale Dünnfilm umfasst vorzugsweise mindestens einen Emulgator. Emulgator ist eine Bezeichnung für Hilfsmittel zur Herstellung und zur Stabilisierung von Emulsionen, die im engeren Sinne auch als grenzflächenaktive Stoffe bzw. Tenside bezeichnet werden können und in der Regel als ölige bis wachsartige, aber auch pulverförmige Stoffe vorliegen. Zur Stabilisierung von Emulsionen über einen längeren Zeitraum werden Hilfsmittel benötigt, die die Entmischung der beiden Phasen Öl und Wasser zum thermodynamisch-stabilen Endzustand unterbinden bzw. so lange verzögern, bis die Emulsion ihre Bestimmung erfüllt hat. Dies kann durch Emulgatoren und/oder Stabilisatoren erreicht werden.

Beispiele für einsetzbare Emulgatoren sind Seifen, Metallseifen, organische Seifen, wie Ethanolaminoleate- oder stearate, sulfurierte Verbindungen, wie Natriumdodecylsulfat, quartäre Ammoniumverbindungen, Fettalkohole, wie Lauryl-, Cetyl-, Stearyl- oder Palmitylalkohol, partielle Fettsäureester mehrwertiger Alkohole mit gesättigten Fettsäuren, wie Glycerolmonostearat, Pentaerythritolmonostearat, Ethylenglycolmonostearat, Propylenglycolmonostearat, partielle Fettsäureester mehrwertiger Alkohole mit ungesättigten Fettsäuren, wie Glycerolmonooleat, Glyceroldioleat, Pentaerythritolmonooleat, ferner Polyoxyethylenester von Fettsäuren, wie Polyoxyethylenstearat, Polymerisationsprodukte aus Ethylenoxid und Propylenoxid mit Fettalkoholen, wie Fettalkoholpolyglycolether oder Fettsäuren, wie Fettsäureethoxylate, Polysorbate, Sorbitanester, Macrogolglycerol-Hydroxystearate, Lecithin, Mono-oder Diglyceride und/oder Polyoxyethylen-Fettsäureether.

Emulgatoren können über den HLB-Wert charakterisiert werden (HLB = hydrophilic-lipophilic balance = hydrolipophiles Verhältnis). Der HLB-Wert ist ein Maß für die Wasser- bzw. Öllöslichkeit von vorwiegend nichtionischen Tensiden und die Stabilität von Emulsionen.

Der HLB-Wert für nichtionische Tenside kann folgendermaßen berechnet werden

$$HLB = 20\ x\ \left(1 - \frac{M_l}{M}\right),$$

wobei $M_l$ die Molmasse des lipophilen Anteils eines Moleküls ist und M die Molmasse des gesamten Moleküls. Der Faktor 20 ist ein frei gewählter Skalierungsfaktor. Es ergibt sich damit eine Skala von 0 bis 20. Ein HLB-Wert von 1 spricht für eine lipophile Verbindung, eine chemische Verbindung mit einem HLB-Wert von 20 hat einen hohen hydrophilen Anteil. Ein Wert zwischen 3 und 8 wird Wasser-in-Öl-Emulgatoren und ein Wert zwischen 8 und 18 Öl-in-Wasser-Emulgatoren zugeordnet.

In einer bevorzugten Ausführungsform ist der orale Dünnfilm dadurch gekennzeichnet, dass der mindestens eine Emulgator einen HLB-Wert von etwa 2 bis 18, bevorzugt von etwa 3 bis 16 aufweist. Emulgatoren mit einem HLB-Wert in diesem Bereich sind besonders gut dazu geeignet, die innere hydrophobe Phase enthaltend den mindestens einen aktiven Wirkstoff aus der Gruppe der Cannabinoide, in der äußeren hydrophilen Phase, enthaltend mindestens ein hydrophiles Polymer, zu stabilisieren. Emulgatoren mit HLB-Werten kleiner als 2 oder größer als 18 haben den Nachteil, dass sie keine stabile Emulsion bilden und somit zu instabilen oralen Dünnfilmen führen.

Besonders bevorzugt umfasst der mindestens eine Emulgator des erfindungsgemäßen oralen Dünnfilms Lecithin, Polysorbat, Sorbitanester, wie Polyoxyethylen(20)-sorbitan-monooleat, Polyoxyethylen(23)-sorbitan-monolaurat, Polyoxyethylen-Fettsäureether, wie Polyoxyethylen-(23)-Laurylether oder Polyoxyethylen-(2)-Stearylalkohol, Macrogolglycerol-Hydroxystearate, Glycerol-Mono- und Dioleate, und/oder Mischungen davon, wie sie beispielsweise unter den Handelsnamen Polysorbat 80, Span 83 bzw. 85, Kolliphor RH40, Tween 20 Tween 80, Atmos 300, Brij S2 und Brij L-23 bekannt sind, ist jedoch nicht auf diese beschränkt.

Der orale Dünnfilm ist dadurch gekennzeichnet, dass Vitamin E in einer Menge von 5 bis 20 Gew.%, bezogen auf das Gesamtgewicht des oralen Dünnfilms, vorliegt.

Weiterhin ist es bevorzugt, dass in einer Ausführungsform des erfindungsgemäßen oralen Dünnfilms in der die Funktion von Vitamin E als zusätzliches stabilisierendes Lösungsmittel wie oben beschrieben nicht erwünscht ist, dennoch mindestens ein zusätzlicher Radikalfänger und/oder ein zusätzliches Antioxidans vorliegt. Der mindestens eine Radikalfänger und/oder das Antioxidans liegt vorzugsweise in einer Menge von deutlich unter etwa 1 Gew.-%, bezogen auf das Gesamtgewicht des oralen Dünnfilms, vor. Der mindestens eine Radikalfänger und/oder das Antioxidans ist vorzugsweise in einer Menge von etwa 0,005 bis etwa 0,3 Gew.-% und bevorzugt von etwa 0,05 bis etwa 0,25 Gew.-%, bezogen auf das Gesamtgewicht des oralen Dünnfilms, in dem erfindungsgemäßen oralen Dünnfilm enthalten.

Bei diesem mindestens einem Radikalfänger und/oder Antioxidans handelt es sich um eine chemische Verbindung, die eine unerwünschte Oxidation anderer Substanzen, speziell des Wirkstoffes, verhindert bzw. vermindert und somit einer Alterung des oralen Dünnfilms entgegen wirkt. Speziell zeichnen sich Radikalfänger und/oder Antioxidantien dadurch aus, dass sie den durch Luftsauerstoff bewirkten oxidativen Abbau empfindlicher Moleküle, hier speziell des enthaltenen Wirkstoffes, verhindern.

Besonders bevorzugt ist der mindestens eine Radikalfänger Butylhydroxyanisol, Ascorbylpalmitat und/oder Butylhydroxytoluol.

Bevorzugt ist der mindestens eine pharmazeutisch aktive Wirkstoff ausgewählt aus der Gruppe der Cannabinoide in dem erfindungsgemäßen oralen Dünnfilm relativ stabil gegenüber Abbau, insbesondere gegenüber oxidativem Abbau.

Es ist bevorzugt, dass nach 2 monatiger Lagerung bei 25°C und 60% relativer Luftfeuchtigkeit (r.F.) gemäß ICH Standards nicht mehr als 15 Gew.-%, bevorzugt nicht mehr als 10 Gew.-% und ganz besonders bevorzugt nicht mehr als 8 Gew.-% des mindestens einen pharmazeutisch aktiven Wirkstoffs ausgewählt aus der Gruppe der Cannabinoide abgebaut wurde.

Dementsprechend ist es bevorzugt, dass nach 2 monatiger Lagerung bei 25°C und 60% relativer Luftfeuchtigkeit (r.F.) gemäß ICH Standards noch 85 Gew.-%, bevorzugt noch 90 Gew.-% und ganz besonders noch 92 Gew.-% des ursprünglich enthaltenen mindestens einen pharmazeutisch aktiven Wirkstoffs ausgewählt aus der Gruppe der Cannabinoide in dem erfindungsgemäßen oralen Dünnfilm enthalten sind.

Der Wirkstoffgehalt wird dabei mittels HPLC-Methode bestimmt.

Außerdem kann der erfindungsgemäße orale Dünnfilm zusätzliche dem Fachmann bekannte Additive enthalten. Diese Additive umfassen beispielsweise Geschmacksstoffe, Farbstoffe, geschmacksmaskierende Stoffe, Permeationsverstärker, Süßstoffe, Füllstoffe, flüssige, vorzugsweise lipophile Hilfsstoffe und/oder pH-Stabilisatoren.

Diese Additive liegen jeweils vorzugsweise in einer Menge von etwa 0,01 bis etwa 10 Gew.-%, bezogen auf das Gesamtgewicht des oralen Dünnfilms, vor.

Weiterhin ist es bevorzugt, dass die äußere hydrophile Phase des erfindungsgemäßen oralen Dünnfilms etwa 30 bis etwa 90 Gew.- %, bevorzugt etwa 30 bis etwa 80 Gew.-%, bezogen auf das Gesamtgewicht des oralen Dünnfilms, ausmacht.

Es ist auch bevorzugt, dass die innere hydrophobe Phase des erfindungsgemäßen oralen Dünnfilms etwa 5 bis etwa 70 Gew.-%, bevorzugt etwa 10 bis etwa 60 Gew.-%, bezogen auf das Gesamtgewicht des oralen Dünnfilms, ausmacht.

Werden die angegebenen Menge über- bzw. unterschritten, so hat das den Nachteil, dass keine homogene Emulsion mehr hergestellt werden bzw. die nötige Menge an Wirkstoff nicht mehr in dem erfindungsgemäßen oralen Dünnfilm untergebracht werden kann.

Der erfindungsgemäße orale Dünnfilm ist vorzugsweise dadurch gekennzeichnet, dass die Menge an Emulgator etwa 1 bis etwa 15 Gew.-%, bevorzugt etwa 2 bis etwa 10 Gew.-%, bezogen auf das Gesamtgewicht des oralen Dünnfilms, beträgt.

Wird die Menge an Emulgator überschritten, so hat das den Nachteil, dass sich die Emulsion physikalisch verändert. Wird die Menge an Emulgator unterschritten, so hat das den Nachteil, dass die hydrophobe Phase nicht in der hydrophilen Phase stabilisiert werden kann.

Der erfindungsgemäße orale Dünnfilm besitzt vorzugsweise eine Fläche von etwa 0.5 cm$^2$ bis etwa 10 cm$^2$, besonders bevorzugt von etwa 2 cm$^2$ bis etwa 8 cm$^2$.

Vorzugsweise weist der erfindungsgemäße orale Dünnfilm ein Flächengewicht von etwa 10 g/m$^2$ bis etwa 300 g/m$^2$, bevorzugt von etwa 20 bis etwa 250 g/m$^2$ und besonders bevorzugt von etwa 50 g/m$^2$ bis etwa 225 g/m$^2$, auf.

Dies entspricht vorzugsweise einer Schichtdicke von etwa 20 $\mu$m bis etwa 500 $\mu$m, bevorzugt von etwa 50 $\mu$m bis etwa 300 $\mu$m, besonders bevorzugt von etwa 100 bis 200 $\mu$m und ganz besonders bevorzugt von etwa 100 bis 150 $\mu$m.

Besonders bevorzugt ist der erfindungsgemäße orale Dünnfilm zudem flexibel, was den Tragekomfort im Mundraum erhöht.

Diese bevorzugte Flexibilität ist insbesondere bei dünnen oralen Dünnfilme mit einer Schichtdicke von etwa 100 bis 200 $\mu$m bzw. von etwa 100 bis 150 $\mu$m ausgeprägt.

Es ist ferner bevorzugt, dass der erfindungsgemäße orale Dünnfilm im Wesentlichen farblos und/oder im Wesentlichen transparent ist.

In einer alternativen, bevorzugten Ausführungsform ist der erfindungsgemäße orale Dünnfilm weiß.

Der erfindungsgemäße orale Dünnfilm löst sich in der Mundhöhle vorzugsweise in einem Zeitraum von weniger als etwa 30 min auf, bevorzugter in einem Zeitraum von weniger als etwa 15 min und ganz besonders bevorzugt in einem Zeitraum von weniger als etwa 10 min.

Bevorzugt umfasst der erfindungsgemäße orale Dünnfilm eine äußere hydrophile Phase, umfassend etwa 50 bis etwa 80 Gew.-%, bevorzugt etwa 65 bis etwa 70 Gew.-%, Polyvinylalkohol, eine innere hydrophobe Phase, umfassend etwa 2 bis etwa 20 Gew.-%, bevorzugt etwa 5 bis etwa 15 Gew.-% Isopropylmyristat, und 5 bis 20 Gew.-%, bevorzugt 5 bis etwa 15 Gew.-% Vitamin E, insbesondere $\alpha$-Tocopherol-acetat und etwa 1 bis etwa 20 Gew.-%, bevorzugt etwa 5 bis etwa 15 Gew.-% mindestens eines Wirkstoffs ausgewählt aus der Gruppe der Cannabinoide, insbesondere Tetrahydrocannabinol, wobei sich die Gew.-% jeweils auf das Gesamtgewicht des oralen Dünnfilms beziehen.

Der Begriff "umfassend", wie hier verwendet, kann auch "bestehend aus" bedeuten.

Die vorliegende Erfindung betrifft ferner ein Verfahren zur Herstellung des erfindungsgemäßen oralen Dünnfilms. Das Verfahren umfasst die Schritte

a1) Herstellen einer wässrigen Lösung bzw. Dispersion, umfassend das mindestens eine hydrophile Polymer;
a2) Herstellen einer Lösung bzw. Dispersion, umfassend mindestens einen pharmazeutisch aktiven Wirkstoff ausgewählt aus der Gruppe der Cannabinoide und die mindestens eine hydrophobe Substanz, wobei die Lösung bzw. Dispersion des Schritts a2) das Vitamin E, in einer Menge von 5 bis 20 Gew.-%, bezogen auf das Gesamtgewicht des oralen Dünnfilms, umfasst, wobei Vitamin E mindestens eine Verbindung ausgewählt aus α-Tocopherol, β-Tocopherol, γ-Tocopherol, δ-Tocopherol, o-Tocotrienol, β-Tocotrienol, γ-Tocotrienol, δ-Tocotrienol und/oder α-Tocopherol-acetat umfasst;

b) Mischen der beiden Lösungen bzw. Dispersionen aus den Schritten a1) und a2), um eine Emulsion zu erhalten; und

c) Ausstreichen und Trocknen der gemäß Schritt b) erhaltenen Emulsion, sodass die getrocknete Emulsion ein Flächengewicht von etwa 50 bis etwa 250 g/m$^2$ aufweist.

Vorzugsweise erfolgt das beschriebene Verfahren unter Schutzgasatmosphäre, vorzugsweise unter Stickstoffatmosphäre, bzw. unter sauerstoffverdrängenden Bedingungen.

Die vorliegende Erfindung betrifft ferner einen oralen Dünnfilm erhältlich nach dem oben beschriebenen Verfahren.

Schließlich betrifft die vorliegende Erfindung die vorstehend näher beschriebenen oralen Dünnfilme und die oralen Dünnfilme, erhältlich nach dem vorstehend beschriebenen Verfahren als Arzneimittel.

Ferner betrifft die vorliegende Erfindung die vorstehend näher beschriebenen oralen Dünnfilme und die oralen Dünnfilme, erhältlich nach dem vorstehend beschriebenen Verfahren zur Verwendung in der Behandlung von Schmerzzuständen, Übelkeit und Erbrechen, neuropathischen Schmerzen, Anorexie, Kachexie, Multipler Sklerose, traumatischen Querschnittserkrankungen, dystonischen Bewegungsstörungen, Asthma bronchiale, epileptischen Anfällen, Entzugssymptomen bei Alkohol-, Benzodiazepin- und Opiatabhängigkeit, Parkinsonerkrankung, Demenzerkrankungen, Alzheimer, Arthritis, Glaukom, Migräne und/oder Dysmenorrhoe.

Offenbart, jedoch nicht beansprucht, sind außerdem Verabreichungsformen für einen Wirkstoff ausgewählt aus der Gruppe der Cannabinoide mit einem Hohlraum, in dem der Wirkstoff vorliegt, wobei die Verabreichungsform wasserlöslich ist, so dass sie sich beim Einnehmen im Mund rasch auflöst und den Wirkstoff ausgewählt aus der Gruppe der Cannabinoide freisetzt. Dadurch, dass der Wirkstoff ausgewählt aus der Gruppe der Cannabinoide in einem Hohlraum der Verabreichungsform eingebracht werden kann, werden technische Beschränkungen für das Einbeziehen größerer Wirkstoffmengen in typische Dünnfilm-Formulierungen umgangen, so dass unproblematisch auch größere Wirkstoffmengen in die Verabreichungsform einbezogen werden können. Zudem ist die thermische Belastung von Wirkstoffen in diesem Verfahren, im Vergleich zur konventionellen Herstellung solcher Verabreichungssysteme, wesentlich geringer, womit eine höhere Lagerzeit erzielt werden kann. Die vorliegende Erfindung betrifft weiterhin Verfahren zur Herstellung entsprechender Verabreichungsformen.

Zur Verabreichung von Wirkstoffen ausgewählt aus der Gruppe der Cannabinoide über die Mundschleimhaut können üblicherweise Buccal- oder Sublingualtabletten verwendet werden, die den Wirkstoff ausgewählt aus der Gruppe der Cannabinoide im Mundraum freisetzen. Die Resorption des Wirkstoffs über die Mundschleimhaut bietet gegenüber anderen peroralen Darreichungsformen einige Vorteile, beispielsweise, dass der Wirkungseintritt aufgrund einer Umgehung der Magen-Darm-Passage rasch erfolgt und dass die Wirkstoffausnutzung hoch ist.

Ein weiteres Problem von Tabletten oder Kapseln besteht darin, dass diese in der Regel geschluckt werden, was bedingt, dass der Patient eine Flüssigkeit bereithält, mit der er diese Darreichungsform zu sich nehmen kann. Teilweise bestehen jedoch bei älteren Patienten oder Kindern Schluckbeschwerden, so dass diese die Einnahme von Tabletten oder Kapseln ablehnen bzw. eine Einnahme nur ungern erfolgt. Zudem ist es möglich, dass Tabletten und Kapseln vom Patienten längere Zeit im Mund behalten und dann ausgespuckt werden. Hieraus resultiert dann nicht selten eine schlechte Compliance, die sich nachteilig beim Heilungsfortschritt bzw. Therapieerfolg bemerkbar macht.

Als alternative Darreichungsform zu den bekannten Buccal- und Sublingualtabletten sind flächenförmige-oblatenartige Darreichungsformen bekannt, die auch als Wafer bezeichnet werden. So beschreibt beispielsweise die US 5,529,782 ein schnell lösliches Filmprodukt aus löslichem Polymermaterial oder komplexen Polysacchariden, das vornehmlich zur Verabreichung von Kontrazeptiva eingesetzt wird. Das Filmprodukt soll eine Dicke von 3 bis 4 mm aufweisen und seine Löslichkeit soll so einstellbar sein, dass es sich innerhalb von 5 bis 60 Sekunden nach Verabreichung aufgelöst hat. Das Filmprodukt kann auch in Form eines Laminats vorliegen, welches mit Gas aufgeschäumte Hohlräume aufweist.

Aus der EP 0 450 141 B1 ist ein Trägermaterial zur Verabreichung von Arzneimitteln bekannt, welches sich bei Kontakt mit Speichel schnell auflöst. Bei diesem Trägermaterial handelt es sich um einen porösen, dehydratisierten, skelettartigen Trägerstoff, insbesondere auf Basis von Proteinen und Polysacchariden. Die durch Dehydratisierung erzeugten Hohlräume werden für das Einbringen von flüssigen Wirkstoffen genutzt.

In der WO 00/18365 wird eine essbare Folie vorgeschlagen, die schnell löslich sein soll, aber auch gut an der Mundschleimhaut haften kann, um antimikrobielle Substanzen abzugeben und die Zahl unerwünschter Mikroorganismen in der Mundflora verringert. Bei den antimikrobiellen Substanzen handelt es sich zum Beispiel um ätherische Öle, die als lipophile Phase vorzugsweise mit Pullulan als Matrixmaterial in der wässrigen Phase vermischt werden.

Die WO 02/02085 beschreibt schnell zerfallende Darreichungsformen zur Freisetzung von Wirkstoffen im Mundraum oder anderen Körperöffnungen, wobei die Darreichungsform eine Matrix aufweist, die zumindest ein wasserlösliches Polymer als Grundsubstanz enthält und die mit Hohlräumen versehen ist.

Auch orale, sich im Mundraum schnell auflösende Filme (OTF (= oral thin film)-Systeme) müssen so formuliert werden, dass der Film bestimmten physikalischen Anforderungen genügt. So müssen derartige Filme beispielsweise eine gewisse Mindestfestigkeit aufweisen, damit sie bei der Handhabung durch den Patienten nicht brechen. Ein weiteres Problem bei OTF-Formulierungen besteht darin, dass die Filme nicht in beliebiger Dicke hergestellt werden können, da die wesentliche Eigenschaft der Filme darin besteht, dass sie sich im Mund rasch auflösen. Dies ist jedoch bei relativ dicken Filmen nicht mehr gewährleistet, da der Zutritt von Wasser oder Speichel in den inneren Bereich des Films bei einer höheren Dicke erschwert ist.

Darüber hinaus sind OTF-Systeme nicht nur in Bezug auf ihre Dicke, sondern auch in Bezug auf ihre maximale Größe beschränkt, da der Anwender den Film ohne Probleme in den Mund, beispielsweise buccal, gingival und auf oder unter die Zunge, legen können soll; dies wäre bei sehr großen Filmen nicht mehr möglich. Aufgrund dieser Rahmenbedingungen ist die zu applizierende Wirkstoffmenge bei normalen filmförmigen OTF-Formulierungen auf etwa 20 mg beschränkt.

Dies stellt zum einen bei Wirkstoffen ein Problem dar, die in höheren Mengen appliziert werden müssen, zum anderen ist dies jedoch auch ein Problem bei bitteren oder anderen als geschmacklich unangenehm empfundenen Wirkstoffen, da diese in der Regel mit signifikanten Mengen an geschmacksmaskierenden Mitteln formuliert werden müssen.

Zudem sind insbesondere die Wirkstoffe aus der Gruppe der Cannabinoide relativ anfällig bezüglich Oxidation und somit relativ instabil, wenn sie grob-dispers in ein wasserlösliches Polymer eingearbeitet werden. Insbesondere Tetrahydrocannabinol (THC) liegt, wenn es in einer hydrophilen Matrix eingebettet ist, in wachsartiger/harziger Form als separate Phase vor, was nachteilig bezüglich dem oxidativen Abbau des Wirkstoffs und der Stabilität des oralen Dünnfilms hinsichtlich Phasentrennung ist. Durch die Empfindlichkeit der Wirkstoffe aus der Gruppe der Cannabinoide gegenüber oxidativem Abbau sind die aus dem Stand der Technik bekannten oralen Dünnfilme insbesondere hinsichtlich ihrer Lagerungsstabilität nachteilig.

Vor diesem Hintergrund besteht ein Bedarf für eine Verabreichungsform von Wirkstoffen, die die gleichen Vorteile wie die bekannten OTF-Formulierungen aufweist, d.h. insbesondere ein rasches Sichauflösen und Freisetzen des Wirkstoffs im Mundraum, andererseits aber keiner so starken Beschränkung in Bezug auf die mögliche zu applizierende Wirkstoffmenge unterliegt.

Ein weiteres Problem bei den bekannten OTF-Systemen besteht darin, dass zur Herstellung der Filme Wirkstoffe mit dem eingesetzten Matrixmaterial vermischt werden müssen, was insbesondere bei den nicht wasserlöslichen Wirkstoffen aus der Gruppe der Cannabinoide besonders schwer möglich ist, wozu entweder ein Lösungsmittel eingesetzt werden kann oder eine Vermischung im Rahmen eines Extrusionsverfahrens erfolgt. Bei einer Verarbeitung mit Hilfe von Lösungsmitteln muss dieses Lösungsmittel im weiteren Verlauf des Verfahrens aus dem System entfernt werden, wozu das System in der Regel erhitzt wird. Dies stellt bei Temperatur labilen Wirkstoffen, wie den Wirkstoffen ausgewählt aus der Gruppe der Cannabinoide ein Problem dar, da sich während der Verdampfung des Lösungsmittels die in das OTF integrierten Wirkstoffe zersetzen/abbauen können. Alternativ kann bei den Lösungen das Lösungsmittel auch unter einem leichten Vakuum entfernt werden. Dies erfordert jedoch geeignete Apparaturen und ist aus technischer Sicht nur mit einem höheren Aufwand zu realisieren, was Kostennachteile mit sich bringt.

Bei einem Extrusionsverfahren werden die Wirkstoffe ebenfalls einer höheren Temperatur ausgesetzt, was zu einer teilweisen Zersetzung des Wirkstoffs führen kann.

Vor diesem Hintergrund besteht auch ein Bedarf für eine Verabreichungsform für einen Wirkstoff ausgewählt aus der Gruppe der Cannabinoide, die hergestellt werden kann, ohne dass der Wirkstoff ausgewählt aus der Gruppe der Cannabinoide hohen Temperaturen ausgesetzt werden muss. Dadurch sollen Verabreichungsformen hergestellt werden können, die auch mit temperaturlabilen Wirkstoffen beladen werden können. Insbesondere soll eine Verabreichungsform, umfassend Wirkstoff ausgewählt aus der Gruppe der Cannabinoide, hergestellt werden, über eine längere Zeit, insbesondere länger als .... Tage gelagert werden können, ohne das der Wirkstoff aus der Gruppe der Cannabinoide signifikant, insbesondere oxidativ, abgebaut wird.

Die Erfindung wird im Folgenden anhand von nicht beschränkenden Beispielen erläutert.

Beispiel 1:

Tabelle 1:

| Zusammensetzung geeigneter oraler Dünnfilme | Substanz | Menge [Gew.-%] |
|---|---|---|
| | Polyvinylalkohol | 70,0 |
| | o-Tocopherol acetat | 10,0 |
| | Isopropylmyristat | 10,0 |
| | Tetrahydrocannabinol | 10,0 |

Orale Dünnfilme mit der in Tabelle 1 aufgeführten Zusammensetzung mit 5,03 mg Dronabinol (THC) als aktivem Wirkstoff und einer Größe von 4,11 cm$^2$ wurden gemäß dem erfindungsgemäßen Verfahren hergestellt und anschließend auf Ihre Stabilität geprüft.

Dazu wurden die oralen Dünnfilme bei 5°C., bei 25°C und 60% r.F. und bei 40°C und 75% r.F. (jeweils nach ICH Standards aus 2003) für 2 Monate gelagert (unter ICH Standards wird dabei die Veröffentlichung "Guidance for Industry Q1A(R2) Stability Testing of New Drug Substances and Products" des U.S. Department of Health and Human Services, Food and Drug Administration, Center for Drug Evaluation and Research (CDER), Center for Biologics Evaluation and Research (CBER), November 2003, ICH, Revision 2, verstanden).

Anschließend wurden der verbleibende Dronabinol-Gehalt sowie die Abbauprodukte von Dronabinol mittels HPLC-Methode bestimmt. Es wurden dabei jeweils 3 unabhängige Tests durchgeführt und der Mittelwert bestimmt.

Die Ergebnisse des Stabilitätstests sind in den Tabellen 2 bis 4 zusammengefasst.

Tabelle 2:

| Ausgangsgehalt an Dronabinol | Gehalt an Dronabinol nach 2 monatiger Lagerung bei | | |
|---|---|---|---|
| | 5°C | 25°C und 60% r. F. | 40°C und 75% r.F. |
| 5,3 mg | 4,97 ± 0,038 mg | 4,89 ± 0,102 mg | 4,99 ± 0,02 mg |
| 100% | 93,8 ± 0,8 % | 92,3 ± 1,93 % | 94,2 ± 0,38 % |

Tabelle 3:

| Ausgangsgehalt an Dronabinol | Gehalt an Dronabinol nach 6 monatiger Lagerung bei | | |
|---|---|---|---|
| | 5°C | 25°C und 60% r. F. | 40°C und 75% r.F. |
| 5,3 mg | 4,80 ± 0,28 mg | 4,80 ± 0,2 mg | 4,90 ± 0,1 mg |
| 100% | 90,6 ± 5,28 % | 90,6 ± 3,77 % | 92,5 ± 1,89 % |

Tabelle 4:

| Lagerungsbedingungen | Abbauprodukte von Dronabinol | | |
|---|---|---|---|
| | Initial | nach 2 Monaten | nach 6 Monaten |
| 5°C | 0,67 % | 0,70% | 0,77% |
| 25°C und 60% r.F | | 1,06% | 1,22% |
| 40°C und 75% r.F | | 1,42% | 1,58% |

Die Stabilitätsuntersuchungen zeigen eine hohe Stabilität des Wirkstoffs Dronabinol in den erfindungsgemäßen oralen Dünnfilmen.

Beispiel 2:

Polymerfilme mit den in Tabelle 6 angegebenen Zusammensetzungen wurden formuliert und mit einer Füllung, wie in Tabelle 6 angegeben, versehen. Hierzu wurden die jeweiligen Polymerfilme zunächst aus Lösungen der genannten Inhaltsstoffe gegossen, die zu einem Film getrocknet wurden. Anschließend wurden entsprechende Filmstücke mit den in Tabelle 1 angegebenen Dimensionen ausgestanzt, zu einer Doppelschicht übereinandergelegt und an drei der Kanten des Films durch Heißsiegelung miteinander verbunden. Anschließend wurde die Füllung eingefüllt und die erhaltene Tasche wurde an der offenen Kante ebenfalls durch Heißsiegelung verschlossen.

Tabelle 5: Die Formulierungen A-C entsprechen nicht den Ansprüchen.

|  | A | B | C | D |
|---|---|---|---|---|
| **Polymerfilm 1** |  |  |  |  |
| Polyvinylalkohol | 96,9 % |  |  | 100 % |
| Polyol N10 |  | 95,9 % |  |  |
| Pullulan |  |  | 95,6 % |  |
| Xanthan |  |  | 0,3 % |  |
| Farbstoff | 0,1 % | 0,1 % | 0,1 % |  |
| Geschmacksstoff | 1 % | 1 % | 1 % |  |
| Süßungsmittel | 2 % | 3 % | 3 % |  |
| Flächengewicht | 45 g/m$^2$ | 45 g/m$^2$ | 45 g/m$^2$ | 50 g/m$^2$ |
| **Polymerfilm 2** | wie Polymerfilm 1 | wie Polymerfilm 1 | wie Polymerfilm 1 | wie Polymerfilm 1 |
| **Füllung** |  |  |  |  |
| THC | 12,5 % | 12,5 % | 12,5 % | 7,7 % |
| Lactose | 83,5 % | 83,5 % |  | 76,9 % |
| Hartfett oder Kakaobutter |  |  | 83,5 % |  |
| Geschmacksstoff | 1 % | 1 % | 1 % |  |
| Süßungsmittel | 3 % | 3 % | 3 % |  |
| Vitamin E |  |  |  | 7,7 % |
| Isopropylmyristat |  |  |  | 7,7 % |
| Menge Füllung | 20 - 80 mg | 20 - 80 mg | 20 - 80 mg | 20-150 mg |
| **Größe Pocket** | 20 x 25 mm | 20 x 25 mm | 20 x 25 mm | 20 x 25 mm |

Es wurden für die Formulierung D der verbleibende Dronabinolgehalt sowie die Abbauprodukte von Dronabinol mittels HPLC-Methode bestimmt. Es wurden dabei jeweils 3 unabhängige Tests durchgeführt und der Mittelwert bestimmt. Die Ergebnisse des Stabilitätstests sind in den Tabellen 6 und 7 zusammengefasst.

Tabelle 6:

| Ausgangsgehalt an Dronabinol | Gehalt an Dronabinol nach 2 monatiger Lagerung bei | | |
|---|---|---|---|
|  | 5°C | 25°C und 60% r.F. | 40°C und 75% r.F. |
| 8,56 mg | 8,03 $\pm$ 1,29 mg | 7,97 $\pm$ 0,96 mg | 8,44 $\pm$ 0,38 mg |
| 100% | 93,8 $\pm$ 15,07 % | 90,6 $\pm$ 11,21 % | 92,5 $\pm$ 4,44 % |

Tabelle 4:

| Lagerungsbedingungen | Abbauprodukte von Dronabinol | |
|---|---|---|
| | Initial | nach 2 Monaten |
| 5°C | 2,47 % | 9,76 % |
| 25°C und 60% r.F | | 8,31 % |
| 40°C und 75% r.F | | 4,47 % |

Die Stabilitätsuntersuchungen zeigen eine hohe Stabilität des Wirkstoffs Dronabinol in den erfindungsgemäßen oralen Dünnfilmen.

## Patentansprüche

1. Oraler Dünnfilm umfassend eine äußere hydrophile Phase, die mindestens ein hydrophiles Polymer enthält, und eine innere hydrophobe Phase, die mindestens eine hydrophobe Substanz und mindestens einen pharmazeutisch aktiven Wirkstoff ausgewählt aus der Gruppe der Cannabinoide enthält, wobei der orale Dünnfilm zusätzlich Vitamin E in einer Menge von 5 bis 20 Gew.-%, bezogen auf das Gesamtgewicht des oralen Dünnfilms, umfasst, wobei Vitamin E mindestens eine Verbindung ausgewählt aus o-Tocopherol, $\beta$-Tocopherol, $\gamma$-Tocopherol, $\delta$-Tocopherol, o-Tocotrienol, $\beta$-Tocotrienol, $\gamma$-Tocotrienol, $\delta$-Tocotrienol und/oder $\alpha$-Tocopherol-acetat umfasst.

2. Oraler Dünnfilm gemäß Anspruch 1, **dadurch gekennzeichnet, dass** der mindestens eine pharmazeutisch aktive Wirkstoff ausgewählt aus der Gruppe der Cannabinoide im Wesentlichen in der inneren hydrophoben Phase vorliegt.

3. Oraler Dünnfilm gemäß irgendeinem der vorhergehenden Ansprüche, **dadurch gekennzeichnet, dass** der mindestens eine pharmazeutisch aktive Wirkstoff ausgewählt aus der Gruppe der Cannabinoide Tetrahydrocannabinol, bevorzugt $\Delta$8-Tetrahydrocannabinol, $\Delta$9-Tetrahydrocannabinol oder R-(6a,10a)-$\Delta$9-Tetrahydrocannabinol, Cannabinol, Cannabidiol, und/oder Cannabichromen ist.

4. Oraler Dünnfilm gemäß einem der vorhergehenden Ansprüche, **dadurch gekennzeichnet**, das die Menge des mindestens einen pharmazeutisch aktive Wirkstoffs ausgewählt aus der Gruppe der Cannabinoide etwa 1 bis etwa 30 Gew.-%, bevorzugt etwa 2 bis etwa 25 Gew.-% und besonders bevorzugt etwa 5 bis etwa 20 Gew.-%, bezogen auf das Gesamtgewicht des oralen Dünnfilms, beträgt.

5. Oraler Dünnfilm gemäß einem der vorhergehenden Ansprüche, **dadurch gekennzeichnet, dass** das mindestens eine hydrophile Polymer in der äußeren hydrophilen Phase ausgewählt ist aus der Gruppe bestehend aus Stärke und Stärkederivaten, Dextran, Cellulose und Cellulosederivaten, wie Carboxymethylcellulose, Hydroxypropylcellulose, Hydroxyethylcellulose, Hydroxypropylmethylcellulose, Hydroxypropylethylcellulose, Natrium-Carboxymethylcellulose, Ethyl- oder Propylcellulose, Polyacrylsäure, Polyacrylat, Polyvinylpyrrolidon, Polyvinylalkohol, Polyethylenoxid-Polymeren, Polyacrylamid, Polyethylenglykol, Gelatine, Kollagen, Alginat, Pectin, Pullulan, Traganth, Chitosan, Alginsäure, Arabinogalactan, Galactomannan, Agar-Agar, Agarose, Carrageen, natürliche Gummen und/oder Copolymeren davon.

6. Oraler Dünnfilm gemäß irgendeinem der vorhergehenden Ansprüche, **dadurch gekennzeichnet, dass** die mindestens eine hydrophobe Substanz in der inneren hydrophoben Phase mittelkettige Triglyceride, Fettsäuren, insbesondere, Isoproylmyristat und/oder Mischungen davon umfasst.

7. Oraler Dünnfilm gemäß irgendeinem der vorhergehenden Ansprüche, **dadurch gekennzeichnet, dass** der orale Dünnfilm zusätzlich mindestens einen Emulgator umfasst.

8. Oraler Dünnfilm gemäß Anspruch 7, **dadurch gekennzeichnet, dass** der mindestens eine Emulgator Polysorbat, Sorbitanester, Polyoxyethylen-Fettsäureether, Macrogolglycerol-Hydroxystearate, Glycerol-Mono- und Dioleate und/oder Mischungen davon umfasst.

9. Oraler Dünnfilm gemäß irgendeinem der vorhergehenden Ansprüche, **dadurch gekennzeichnet, dass** die äußere hydrophile Phase 30 bis 80 Gew.-%, bezogen auf das Gesamtgewicht des oralen Dünnfilms, ausmacht.

10. Oraler Dünnfilm gemäß irgendeinem der vorhergehenden Ansprüche, **dadurch gekennzeichnet, dass** die innere hydrophobe Phase etwa 10 bis etwa 60 Gew.-%, bezogen auf das Gesamtgewicht des oralen Dünnfilms, ausmacht.

11. Oraler Dünnfilm gemäß irgendeinem der vorhergehenden Ansprüche, **dadurch gekennzeichnet, dass** die Menge an Emulgator etwa 2 bis etwa 10 Gew.-%, bezogen auf das Gesamtgewicht des oralen Dünnfilms, beträgt.

12. Oraler Dünnfilm gemäß irgendeinem der vorhergehenden Ansprüche, **dadurch gekennzeichnet, dass** nach 2 monatiger Lagerung bei 25°C und 60% relativer Luftfeuchtigkeit noch mindestens 85 Gew.-%, des ursprünglich enthaltenen mindestens einen pharmazeutisch aktiven Wirkstoffs ausgewählt aus der Gruppe der Cannabinoide in dem erfindungsgemäßen oralen Dünnfilm enthalten sind.

13. Verfahren zur Herstellung eines oralen Dünnfilms gemäß irgendeinem der Ansprüche 1 bis 12, umfassend die Schritte:

a1) Herstellen einer wässrigen Lösung bzw. Dispersion, umfassend das mindestens eine hydrophile Polymer;
a2) Herstellen einer Lösung bzw. Dispersion, umfassend mindestens einen pharmazeutisch aktiven Wirkstoff ausgewählt aus der Gruppe der Cannabinoide und die mindestens eine hydrophoben Substanz,
wobei die Lösung bzw. Dispersion des Schritts a2) das Vitamin E , in einer Menge von 5 bis 20 Gew.-%, bezogen auf das Gesamtgewicht des oralen Dünnfilms, umfasst, wobei Vitamin E mindestens eine Verbindung ausgewählt aus $\alpha$-Tocopherol, $\beta$-Tocopherol, $\gamma$-Tocopherol, $\delta$-Tocopherol, $\alpha$-Tocotrienol, $\beta$-Tocotrienol, $\gamma$-Tocotrienol, $\delta$-Tocotrienol und/oder o-Tocopherol-acetat umfasst;
b) Mischen der beiden Lösungen bzw. Dispersionen aus den Schritten a1) und a2), um eine Emulsion zu erhalten; und
c) Ausstreichen und Trocknen der gemäß Schritt b) erhaltenen Emulsion, sodass die getrocknete Emulsion ein Flächengewicht von etwa 20 bis 250 g/m$^2$ aufweist.

14. Oraler Dünnfilm erhältlich nach dem Verfahren gemäß Anspruch 13.

15. Oraler Dünnfilm gemäß den Ansprüchen 1 bis 12 und 14 zur Verwendung als Arzneimittel, insbesondere in der Behandlung von Schmerzzuständen, Übelkeit und Erbrechen, neuropathischen Schmerzen, Anorexie, Kachexie, Multipler Sklerose, traumatischen Querschnittserkrankungen, dystonischen Bewegungsstörungen, Asthma bronchiale, epileptischen Anfällen, Entzugssymptomen bei Alkohol-, Benzodiazepin- und Opiatabhängigkeit, Parkinsonerkrankung, Demenzerkrankungen, Alzheimer, Arthritis, Glaukom, Migräne, Dysmenorrhoe.

## Claims

1. An oral thin film comprising an outer hydrophilic phase, which contains at least one hydrophilic polymer, and an inner hydrophobic phase, which contains at least one hydrophobic substance, and at least one pharmaceutically active substance selected from the group of cannabinoids, wherein the oral thin film additionally comprises vitamin E in an amount from 5 to 20 % by weight, in relation to the total weight of the oral thin film, wherein vitamin E comprises at least one component selected from $\alpha$-tocopherol, $\beta$-tocopherol, $\gamma$-tocopherol, $\delta$-tocopherol, $\alpha$-tocotrienol, $\beta$-tocotrienol, $\gamma$-tocotrienol, 5-tocotrienol and/or $\alpha$-tocopherol acetate.

2. The oral thin film according to claim 1, **characterised in that** the at least one pharmaceutically active substance selected from the group of cannabinoids is present substantially in the inner hydrophobic phase.

3. The oral thin film according to any one of the preceding claims, **characterised in that** the at least one pharmaceutically active substance selected from the group of cannabinoids is tetrahydrocannabinol, preferably $\Delta$8-tetrahydrocannabinol, $\Delta$9-tetrahydrocannabinol or R-(6a,10a)-$\Delta$9-tetrahydrocannabinol, cannabinol, cannabidiol, and/or cannabichromene.

4. The oral thin film according to one of the preceding claims, **characterised in that** the amount of the at least one pharmaceutically active substance selected from the group of cannabinoids is about 1 to about 30 % by weight, preferably about 2 to about 25 % by weight, and especially preferably about 5 to about 20 % by weight, in relation to the total weight of the oral thin film.

5. The oral thin film according to one of the preceding claims, **characterised in that** the at least one hydrophilic polymer in the outer hydrophilic phase is selected from the group consisting of starch and starch derivatives, dextran, cellulose and cellulose derivatives, such as carboxymethyl cellulose, hydroxypropyl cellulose, hydroxyethyl cellulose, hydroxypropylmethyl cellulose, hydroxypropyl ethyl cellulose, sodium carboxymethyl cellulose, ethyl or propyl cellulose, polyacrylic acid, polyacrylate, polyvinyl pyrrolidone, polyvinyl alcohol, polyethylene oxide polymers, polyacrylamide, polyethylene glycol, gelatine, collagen, alginate, pectin, pullulan, tragacanth, chitosan, alginic acid, arabinogalactan, galactomannan, agar-agar, agarose, carrageenan, natural gums and/or copolymers thereof.

6. The oral thin film according to any one of the preceding claims, **characterised in that** the at least one hydrophobic substance in the inner hydrophobic phase comprises medium-chain triglycerides, fatty acids, especially isopropyl myristate and/or mixtures thereof.

7. The oral thin film according to any one of the preceding claims, **characterised in that** the oral thin fil additionally comprises at least one emulsifier.

8. The oral thin film according to claim 7, **characterised in that** the at least one emulsifier comprises polysorbate, sorbitan esters, polyoxyethylene fatty acid ethers, macrogol glycerol hydroxy stearates, glycerol mono- and *dioleates* and/or mixtures thereof.

9. The oral thin film according to any one of the preceding claims, **characterised in that** the outer hydrophilic phase constitutes 30 to 80 % by weight in relation to the total weight of the oral thin film.

10. The oral thin film according to any one of the preceding claims, **characterised in that** the inner hydrophobic phase constitutes about 10 to about 60 % by weight in relation to the total weight of the oral thin film.

11. The oral thin film according to any one of the preceding claims, **characterised in that** the amount of emulsifier is about 2 to about 10 % by weight in relation to the total weight of the oral thin film.

12. The oral thin film according to any one of the preceding claims, **characterised in that**, after storage for 2 months at 25°C and 60% relative humidity, at least 85 % by weight of the originally contained at least one pharmaceutically active substance selected from the group of cannabinoids is still contained in the oral thin film according to the invention.

13. A method for producing an oral thin film according to any one of claims 1 to 12, comprising the steps of:

al) producing an aqueous solution or dispersion comprising the at least one hydrophilic polymer;
a2) producing a solution or dispersion comprising at least one pharmaceutically active substance selected from the group of cannabinoids and the at least one hydrophobic substance, wherein the solution or dispersion of step a2) comprises vitamin E in an amount from about 5 to about 20 % by weight, in relation to the total weight of the oral thin film, wherein vitamin E comprises at least one component selected from $\alpha$-tocopherol, $\beta$-tocopherol, $\gamma$-tocopherol, $\delta$-tocopherol, $\alpha$-tocotrienol, $\beta$-tocotrienol, $\gamma$-tocotrienol, 5-tocotrienol and/or $\alpha$-tocopherol acetate;
b) mixing the two solutions or dispersions from steps a1) and a2) to obtain an emulsion; and
c) spreading and drying the emulsion obtained in step b) so that the dried emulsion has a weight per unit area of about 20 to 250 g/m$^2$.

14. An oral thin film obtainable by the method according to claim 13.

15. The oral thin film according to claims 1 to 12 and 14 for the use as a medicament, especially in the treatment of pain conditions, nausea and vomiting, neuropathic pain, anorexia, cachexia, multiple sclerosis, traumatic paraplegia, dystonic movement disorders, bronchial asthma, epileptic seizures, withdrawal symptoms of alcohol, benzodiazepine and opiate dependence, Parkinson's disease, dementia, Alzheimer's disease, arthritis, glaucoma, migraine, dysmenorrhoea.

**Revendications**

1. Film mince à prise orale comprenant une phase hydrophile extérieure qui contient au moins un polymère hydrophile, et une phase hydrophobe intérieure qui contient au moins une substance hydrophobe et au moins un principe actif pharmaceutiquement sélectionné à partir du groupe des cannabinoïdes, dans lequel le film mince à prise orale comprend en outre de la vitamine E dans une quantité de 5 à 20 % en poids, par rapport au poids total du film mince oral, dans lequel la vitamine E comprend un composé sélectionné à partir de $\alpha$-tocophérol, $\beta$-tocophérol, $\gamma$-tocophérol, 5-tocophérol, $\alpha$-tocotriénol, $\beta$-tocotriénol, $\gamma$-tocotriénol, $\delta$-tocotriénol et/ou acétate de $\alpha$-tocophérol.

2. Film mince à prise orale selon la revendication 1, **caractérisé en ce que** l'au moins un principe actif pharmaceutiquement sélectionné à partir du groupe des cannabinoïdes se présente sensiblement dans la phase hydrophobe intérieure.

3. Film mince à prise orale selon l'une quelconque des revendications précédentes, **caractérisé en ce que** l'au moins un principe actif pharmaceutiquement sélectionné à partir du groupe des cannabinoïdes est du tétrahydrocannabinol, de préférence $\Delta$8-tétrahydrocannabinol, $\Delta$9-tétrahydrocannabinol ou R-(6a, 10a)-$\Delta$9-tétrahydrocannabinol, cannabinol, cannabidiol et/ou des cannabichromènes.

4. Film mince à prise orale selon l'une des revendications précédentes, **caractérisé en ce que** la quantité de l'au moins un principe actif pharmaceutiquement sélectionné à partir du groupe des cannabinoïdes est comprise entre environ 1 et environ 30 % en poids, de préférence entre environ 2 et environ 25 % en poids et de manière particulièrement préférée entre environ 5 et environ 20 % en poids, par rapport au poids total du film mince à prise orale.

5. Film mince à prise orale selon l'une des revendications précédentes, **caractérisé en ce que** l'au moins un polymère hydrophile dans la phase hydrophile extérieure est sélectionné à partir du groupe se composant d'amidon et dérivés d'amidon, dextrane, cellulose et dérivés de cellulose tels que carboxyméthylcellulose, hydroxypropylcellulose, hydroxyéthylcellulose, hydroxypropylméthylcellulose, hydroxypropyléthylcellulose, carboxyméthylcellulose de sodium, éthyl- ou propylcellulose, acide polyacrylique, polyacrylate, polyvinylpyrrolidone, alcool polyvinylique, polymères de polyéthylèneoxyde, polyacrylamide, polyéthylèneglycol, gélatine, collagène, alginate, pectine, pullulane, gomme adragante, chitosane, acide alginique, arabinogalactane, galactomannane, agar-agar, agarose, carragheen, gommes naturelles et/ou copolymères de ceux-ci.

6. Film mince à prise orale selon l'une quelconque des revendications précédentes, **caractérisé en ce que** l'au moins une substance hydrophobe dans la phase hydrophobe intérieure comprend des triglycérides à mi-chaîne, acides gras, en particulier myristate d'isopropyle et/ou des mélanges de ceux-ci.

7. Film mince à prise orale selon l'une quelconque des revendications précédentes, **caractérisé en ce que** le film mince à prise orale comprend en outre au moins un émulsifiant.

8. Film mince à prise orale selon la revendication 7, **caractérisé en ce que** l'au moins un émulsifiant comprend du polysorbate, ester de sorbitanne, polyoxyéthylène-éther d'acide gras, hydroxystéarate de macrogolglycérol, monoet di-oléate de glycérol et/ou des mélanges de ceux-ci.

9. Film mince à prise orale selon l'une quelconque des revendications précédentes, **caractérisé en ce que** la phase hydrophile extérieure constitue 30 à 80 % en poids, par rapport au poids total du film mince à prise orale.

10. Film mince à prise orale selon l'une quelconque des revendications précédentes, **caractérisé en ce que** la phase hydrophobe intérieure constitue environ 10 à environ 60 % en poids, par rapport au poids total du film mince à prise orale.

11. Film mince à prise orale selon l'une quelconque des revendications précédentes, **caractérisé en ce que** la quantité d'émulsifiant est comprise entre environ 2 et environ 10 % en poids, par rapport au poids total du film mince.

12. Film mince à prise orale selon l'une quelconque des revendications précédentes, **caractérisé en ce qu'**après un stockage de 2 mois à 25 °C et à 60 % d'humidité d'air relative, encore au moins 85 % en poids de l'au moins un principe actif pharmaceutiquement contenu à l'origine sélectionné à partir du groupe des cannabinoïdes sont contenus dans le film mince à prise orale selon l'invention.

13. Procédé de fabrication d'un film mince à prise orale selon l'une quelconque des revendications 1 à 12, comprenant les étapes suivantes :

a1) la fabrication d'une solution ou dispersion aqueuse, comprenant l'au moins un polymère hydrophile ;

a2) la fabrication d'une solution ou dispersion, comprenant au moins un principe actif pharmaceutiquement sélectionné à partir du groupe des cannabinoïdes et l'au moins une substance hydrophobe, dans lequel la solution ou la dispersion de l'étape a2) comprend de la vitamine E, dans une quantité de 5 à 20 % en poids, par rapport au poids total du film mince à prise oral, dans lequel de la vitamine E comprend au moins un composé sélectionné à partir de $\alpha$-tocophérol, $\beta$-tocophérol, $\gamma$-tocophérol, $\delta$-tocophérol, $\alpha$-tocotriénol, $\beta$-tocotriénol, $\gamma$-tocotriénol, $\delta$-tocotriénol et/ou acétate de $\alpha$-tocophérol ;

b) le mélange des deux solutions ou dispersions des étapes a1) et a2) afin d'obtenir une émulsion ; et

c) l'étalement et le séchage de l'émulsion obtenue selon l'étape b) de sorte que l'émulsion séchée présente un grammage d'environ 20 à 250 g/m$^2$.

14. Film mince à prise orale pouvant être obtenu selon le procédé selon la revendication 13.

15. Film mince à prise orale selon les revendications 1 à 12 et 14 pour l'utilisation comme médicament, en particulier dans le traitement d'états douloureux, nausée et vomissement, douleurs neuropathiques, anorexie, cachexie, sclérose en plaques, lésions traumatiques de la moelle épinière, troubles dystoniques du mouvement, asthme bronchique, crises d'épilepsie, symptômes de sevrage en cas de dépendance à l'alcool, aux benzodiazépines et aux opiacés, maladie de Parkinson, formes de démence, maladie d'Alzheimer, arthrite, glaucome, migraines, dysménorrhée.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 03105800 A2
- CA 2922959 A1
- WO 02064109 A
- US 5529782 A
- EP 0450141 B1
- WO 0018365 A
- WO 0202085 A

**In der Beschreibung aufgeführte Nicht-Patentliteratur**

- Guidance for Industry Q1A(R2) Stability Testing of New Drug Substances and Products. U.S. Department of Health and Human Services, Food and Drug Administration, Center for Drug Evaluation and Research (CDER), Center for Biologics Evaluation and Research (CBER), November 2003